# EUROPEAN PATENT SPECIFICATION

(11) **EP 0 694 063 B1**
(45) Date of publication and mention of the grant of the patent: **28.04.2004**
(21) Application number: 94907508.9
(22) Date of filing: 18.02.1994
(51) Int. Cl.: C12N 9/00

(54) **AN ALPHA-AMYLASE FROM PYROCOCCUS**
DAS STÄRKEABBAUENDES ENZYM ALPHA-AMYLASE AUS PYROCOCCUS
UNE ALPHA-AMYLASE DE PYROCOCUS

(30) Priority: 19.02.1993 DK 19693; 13.09.1993 DK 102793; 03.11.1993 DK 124593
(43) Date of publication of application: 31.01.1996
(73) Proprietor: Novozymes A/S, 2880 Bagsvaerd (DK)
(72) Inventor: JORGENSEN, Steen Troels, DK-3450 Aller d (DK)
(86) International application number: PCT/DK1994/000071
(87) International publication number: WO 1994/019454

(56) References cited:
- WO-A-90/11352
- WO-A-93/10248
- Dialog Information Services, File 5, BIOSIS, Dialog Accession No. 8615400, Biosis Accession No. 92080400, KOCH R. et al.: "Purification and Properties of a Hyperthermoactive Alpha Amylase from the Archaeobacterium Pyrococcus-Woesei", Arch. Microbiol. 155 (6), 1991, 572-578.

## Description

### FIELD OF THE INVENTION

The present invention relates to a DNA construct comprising a DNA sequence encoding an amylolytic enzyme, in particular a *Pyrococcus* α-amylase or a variant thereof, and vector and cell harbouring the DNA construct. Furthermore, the invention relates to a process for producing the amylolytic enzyme by use of recombinant DNA techniques.

### BACKGROUND OF THE INVENTION

During the last decade enzymes produced by thermophilic microorganisms such as archaebacteria have been the subject of increasing interest mainly because of their high thermostability which, for a number of industrial applications, is desirable.

Examples of such hyperthermophilic enzymes are those produced by strains of the thermophilic archaebacterium *Pyrococcus*. For instance, WO 90/11352 discloses novel *Pyrococcus* alpha-amylases obtained from strains of the species *P. woesei* and *P. furiosus* by conventional fermentation procedures involving culturing of the *Pyrococcus* strains and isolating alpha-amylase preparations therefrom. Furthermore, Koch et al. (1990) and Brown et al. (1990) describe partially purified *P. furiosus* α-amylase. Koch et al. (1991) describe the purification and properties of an alpha-amylase from Pyrococcus woesei. WO 92/02614 discloses novel thermostable pullulanases obtained from *Pyrococcus spp*.

The *Pyrococcus* α-amylases and pullulanases disclosed in WO 90/11352 and WO 92/02614, respectively, have been found to possess an extremely high thermostability as compared to other known α-amylases and pullulanases, and are stated to be useful in high-temperature processes involving α-amylase or pullulanase activity.

It would be desirable to facilitate the production of such amylolytic enzymes, and amylolytic enzymes in general, both with a view to improve the purity thereof and with a view to provide larger amounts of the purified enzyme at lower cost than what is possible by cultivation of a parent strain capable of expressing the enzyme.

### BRIEF DISCLOSURE OF THE INVENTION

The present inventor have now succeeded in cloning a DNA sequence encoding an α-amylase from a strain of the archebacterial species *Pyrococcus furiosus* and obtaining α-amylase expression from a host cell harbouring said DNA sequence. The present invention is based on this finding.

More specifically, in a first aspect the invention relates to a DNA construct comprising a DNA sequence encoding a *Pyrococcus* α-amylase shown in SEQ ID No. 1 or a DNA sequence encoding an alpha-amylase being at least 70% identical with the DNA sequence shown in SEQ ID NO: 1.

In a still further aspect the present invention relates to a vector harbouring the DNA construct of the invention, and a cell which either harbours the DNA construct or the vector of the invention.

In the course of the research leading to the present invention it was surprisingly found to be possible to obtain α-amylase expression from a strain of *Bacillus* transformed with a *Pyrococcus* DNA sequence without any modification of said DNA sequence being required. Such modification which, e.g. involves modification or replacement of the ribosome binding site of the gene to be translated, is normally considered to be a prerequisite for obtaining an efficient translation and thereby expression from non-gram positive DNA sequences in *Bacillus*, cf. also the explanation given in Example 6 hereinafter. As far as the present inventors are aware there have been no prior disclosure of the expression of *Pyrococcus* α-amylase genes in *Bacillus*. WO 93/10248, which was published only after the priority date of the present application, discloses the use of *Bacillus licheniformis* as a host cell for the recombinant production of certain proteins including *Pyrococcus* α-amylase.

Based on the above described finding it is contemplated that direct expression of *Pyrococcus* α-amylase genes, in general, may be obtained in a strain of *Bacillus* and accordingly, in a particular aspect the invention relates to a recombinant *Bacillus* cell, which is different from a cell of *Bacillus licheniformis*, and which harbours a DNA construct comprising a DNA sequence encoding a *Pyrococcus* α-amylase or a variant thereof, the DNA construct optionally being present on an expression vector.

In a further aspect the present invention relates to a process for the production of a recombinant amylolytic enzyme, in particular a recombinant *Pyrococcus* α-amylase or variant thereof, comprising culturing a cell as described above in a suitable culture medium under conditions permitting expression of the amylolytic enzyme, and recovering the resulting amylolytic enzyme from the culture.

By use of the process of the invention it is contemplated that an amylolytic enzyme, such as a *Pyrococcus* α-amylase or a variant thereof, encoded by the DNA construct of the invention, may be produced in high amounts and in a high purity, thereby, for instance, being able to optimize the production of the amylolytic enzyme in question in mono-component form essentially free from any other enzymatic activities.

It is contemplated that the *Pyrococcus* α-amylase encoded by the DNA construct of the invention is particularly suitable for use in starch liquefaction carried out at high temperatures. Accordingly, in a still further aspect the invention relates to a starch liquefaction process which comprises subjecting an aqueous starch slurry to enzymatic liquefaction in the presence of a *Pyrococcus* α-amylase or a variant of the invention.

In a final aspect the invention relates to the use of an amylolytic enzyme of the invention for starch modification, including starch liquefaction and/or saccharification, debranching of starch, production of various syrups, e.g. maltose syrup, production of cyclodextrin, and production of oligosaccharides.

### DETAILED DESCRIPTION OF THE INVENTION

The partial DNA sequences apparent from SEQ ID Nos. 2, 3, 4, 5 and 6 were derived from genomic clones prepared from a strain of the archaebacterial species *Pyrococcus furiosus* harbouring a DNA sequence of about 5 kb which encodes an α-amylase activity, cf. the examples hereinafter. The partial DNA sequences SEQ ID Nos. 3 and 6, which were identified in different clones, are identical except for the sequence SEQ ID No. 6 being longer than the sequence SEQ ID No. 3. Both of the sequences comprises the N-terminal part of the coding sequence of the α-amylase starting at position 7 (GTG). SEQ ID Nos. 2 and 4 constitute internal parts of the coding sequence shown in SEQ ID NO. 1 starting in the same XbaI site (TCTAGA). SEQ ID No. 2 is read in the upstream direction, whereas SEQ ID No. 4 is read in the downstream direction. The SEQ ID No. 5 is located about 3.3 kb downstream of the 3' end of the coding sequence and is read upstream.

The partial DNA sequences (SEQ ID Nos. 2, 3, 4, 5 and 6) may either separately or in combinations of two or more be used in the isolation of DNA sequences encoding a *Pyrococcus* α-amylase, cf. the examples herein. Thus, these DNA sequences have been found to constitute important tools in the isolation of the DNA sequence identified in SEQ ID No. 1 and thus in the preparation of recombinant *Pyrococcus* α-amylase.

In the DNA construct of the invention encoding a *Pyrococcus* α-amylase or a variant thereof, the analogue of the DNA sequence shown in SEQ ID No. 1 or of any of the partial DNA sequences shown in SEQ ID Nos. 2, 3, 4, 5 and 6 may, for instance, be a subsequence of any of these DNA sequences, a genetically engineered modification of said sequences which may be prepared by well-known procedures, e.g. by site-directed mutagenesis, or a DNA sequence encoding a *Pyrococcus* α-amylase or a variant thereof isolated from a strain of a *Pyrococcus* spp. In any event the analogous DNA sequence should hybridize to an oligonucleotide probe which may be prepared on the basis of the DNA sequence shown in SEQ ID No. 1 or of any of the partial DNA sequences SEQ ID Nos. 2, 3, 4, 5 or 6, e.g. constituting a subsequence or the entire sequence thereof. Alternatively, a suitable oligonucleotide probe may be prepared on the basis of an amino acid sequence encoded by a DNA construct of the invention harbouring one or more of the DNA sequence SEQ ID Nos. 1, 2, 3, 4, 5, and 6, e.g. on the basis of any part of the amino acid sequences shown in SEQ ID Nos. 8 or 9.

On the basis of the amino acid sequence shown in SEQ ID No. 9 of a mature α-amylase enzyme, an analysis has been made of the extent to which the DNA sequence shown in SEQ ID No. 1 may vary without effecting it's α-amylase encoding capability. As a result of this analysis it has been found that the most "different" DNA sequence having retained the capability of encoding the polypeptide having the amino acid sequence shown in SEQ ID No. 9 is one which shows a homology of about 62.6% with the DNA sequence shown in SEQ ID No. 1.

Accordingly, the analogue of the DNA sequence shown in SEQ ID No. 1 is preferably one being at least 62.5% homologous to the said DNA sequence, more preferably at least 70% homologous, such as at least 80% homologous, still more preferably at least 90% homologous with the DNA sequence shown in SEQ ID No. 1. In the present context the homology is determined as the degree of identity between the two sequences indicating a derivation of the first sequence from the second. In a particular embodiment, the analogue of the DNA sequence shown in SEQ ID No. 1 is a synthetic gene.

The hybridization of a DNA sequence with the relevant oligonucleotide probe(s) may be carried out under any suitable conditions allowing the DNA sequences to hybridize. For instance, such conditions are hybridization under specified conditions, e.g. involving presoaking in 5xSSC and prehybridizing for 1h at ∼40°C in a solution of 20% formamide, 5xDenhardt's solution, 50mM sodium phosphate, pH 6.8, and 50µg of denatured sonicated calf thymus DNA, followed by hybridization in the same solution supplemented with 100µM ATP for 18h at ∼40°C, or other methods described by e.g. Sambrook et al., 1989.

As stated above, the DNA sequences shown in SEQ ID Nos. 1-6 were determined from a genomic clone prepared from a strain of *Pyrococcus furiosus*, more specifically from the *P*. *furiosus* strain DSM 3638 available from the Deutsche Sammlung von Mikroorganismen und Zellkulturen GmbH. Due to the high degree of homology normally found within the group of amylolytic enzymes it is contemplated that DNA sequences, which are homologous to the DNA sequences shown in SEQ ID Nos. 1-6 and which encode amylolytic enzymes other than the *Pyrococcus* α-amylase disclosed herein, may be identified in other organisms, including thermophilic organisms such as archaebacteria and other types of organisms living under extreme conditions.

Accordingly, the DNA sequence of a DNA construct of the invention may be derived from an archaebacterium, in particular a thermophilic archaebacterium such as a strain of the genus *Pyrococcus*, especially from a strain of *P. woesei* or *P*. *furiosus*. Such strains may be isolated by established procedures from places expected to harbour archaebacteria, e.g. hot springs or the like, or may be obtained from publicly available culture collections.

An example of an analogous DNA sequence is a DNA sequence derivable from the *P. woesei* strain DSM 3773 available from the Deutsche Sammlung von Mikroorganismen und Zellkulturen GmbH. Chromosomal DNA isolated from this strain has been found to hybridize with the 4.5 kb genomic fragment containing the α-amylase gene from *P. furiosus* (cf. examples 3 and 4). Furthermore, it is contemplated that analogous DNA sequences of the DNA construct of the invention are derivable from mutants and derivatives of the deposited *P. furiosus* strain DSM 3638 and *P*. *woesei* strain DSM 3773 having retained their α-amylase producing capability.

In the DNA construct of the invention comprising a DNA sequence encoding an amylolytic enzyme, the homology with the amino acid sequence shown in SEQ ID No. 9, is intended to indicate the degree of identity between the polypeptide encoded by the DNA sequence and the amino acid sequence shown in SEQ ID No. 9 indicating a derivation of the first sequence from the second. In particular, a polypeptide is considered to be homologous to the amino acid sequence shown in SEQ ID No. 9 if a comparison of the respective amino acid sequences reveals an identity of greater than about 70% such as greater than about 75%, 80%, 85%, 90% or even 95%. Sequence comparisons can be performed via known algorithms, such as the one described by Lipman and Pearson (1985).

A preferred example of a DNA construct of the invention encoding an amylolytic enzyme is a DNA construct encoding a *Pyrococcus* α-amylase or a variant thereof as described above.

The DNA sequence of the DNA constructs of the invention may be prepared by well-known methods. Thus, the DNA sequence may, for instance, be isolated by establishing a cDNA or genomic library from an organism expected to harbour the sequence, e.g. a cell as described above, and screening for positive clones by conventional procedures. Examples of such procedures are hybridization to oligonucleotide probes as described above in accordance with standard techniques (cf. Sambrook et al., 1989), and/or selection for clones expressing amylolytic, such as α-amylase activity, and/or selection for clones producing a protein which is reactive with an antibody raised against an amylolytic enzyme such as a *Pyrococcus* α-amylase encoded by the DNA construct of the invention.

A preferred method of isolating a DNA construct of the invention from a cDNA or genomic library is by use of polymerase chain reaction (PCR) using degenerate oligonucleotide probes prepared on the basis of any of the DNA sequences shown in SEQ ID Nos. 2-6 or of an amino acid sequence encoded by a DNA construct of the invention, e.g. an amino acid sequence as shown in SEQ ID No. 8 or 9 or any of the partial amino acid sequences (a)-(R) disclosed above. For instance, the PCR may be carried out using the techniques described in US Patent No. 4,683,202 or by R.K. Saiki et al. (1988).

Alternatively, the DNA sequence of the DNA construct of the invention may be prepared synthetically by established standard methods, e.g. the phosphoamidite method described by Beaucage and Caruthers (1981), or the method described by Matthes et al. (1984). According to the phosphoamidite method, oligonucleotides are synthesized, e.g. in an automatic DNA synthesizer, purified, annealed, ligated and cloned in appropriate vectors.

Finally, the DNA construct may be of mixed genomic and synthetic, mixed synthetic and cDNA or mixed genomic and cDNA origin prepared by ligating fragments of synthetic, genomic or cDNA origin (as appropriate), the fragments corresponding to various parts of the entire recombinant DNA molecule, in accordance with standard techniques.

As stated above, the DNA constructs of the invention may also comprise a genetically modified DNA sequence. Such sequence may be prepared on the basis of a genomic or cDNA sequence encoding an amylolytic, such as α-amylase activity, suitably modified at a site corresponding to the site(s) of the polypeptide at which it is desired to introduce amino acid substitutions, e.g. by site-directed mutagenesis using synthetic oligonucleotides encoding the desired amino acid sequence for homologous recombination in accordance with well-known procedures, or by use of random mutagenesis, e.g. through radiation, chemical treatment or PCR using random oligonucleotide primers.

Examples of suitable modifications of the DNA sequence are nucleotide substitutions which do not give rise to another amino acid sequence of the polypeptide, but which may correspond to the codon usage of the host organism into which the recombinant DNA molecule is introduced, or nucleotide substitutions which do give rise to a different amino acid sequence and therefore, possibly, a different polypeptide structure without, however, impairing the essential properties of the polypeptide related to amylolytic activity and/or immunologically cross-reactivity as described above. Other examples of possible modifications are insertion of one or more nucleotides into the sequence, addition of one or more nucleotides at either end of the sequence and deletion of one or more nucleotides at either end of or within the sequence.

The vector carrying a DNA construct of the invention, which is preferably a recombinant expression vector, may be any vector which may conveniently be subjected to recombinant DNA procedures, and the choice of expression vector will often depend on the host cell into which it is to be introduced. Thus, the vector may be an autonomously replicating vector, i.e. a vector which exists as an extrachromosomal entity, the replication of which is independent of chromosomal replication, e.g. a plasmid or a bacteriophage. Alternatively, the vector may be one which, when introduced into a host cell, is integrated into the host cell genome and replicated together with the chromosome (s) into which it has been integrated.

In the DNA construct or the vector, the DNA sequence should be operably connected to a suitable promoter sequence. The promoter may be any DNA sequence which shows transcriptional activity in the host cell of choice and may be derived from genes encoding proteins either homologous or heterologous to the host cell. Examples of suitable promoters for directing the transcription of the DNA construct of the invention are the promoter of the lac operon of *E*.*coli*, the *Streptomyces coelicolor* agarase gene *dagA* promoters, the promoters of the *Bacillus licheniformis* α-amylase gene (*amy*L), the promoters of the *Bacillus stearothermophilus* maltogenic amylase gene (*amy*M), the promoters of the *Bacillus Amyloliquefaciens* α-amylase (*amy*Q), etc.

The DNA construct and/or expression vector of the invention may also comprise a suitable terminator operably connected to the DNA sequence encoding the amylolytic enzyme such as the *Pyrococcus* α-amylase or variant thereof of the invention. An example of a suitable terminator is that of the *Bacillus licheniformis* α-amylase gene.

The DNA construct and/or vector may further comprise a DNA sequence enabling the vector to replicate in the host cell in question. Examples of such sequences are the origins of replication of plasmids pUC19, pACYC177, pUB110, pE194, pAMB1 and pIJ702.

The DNA construct and/or vector may also comprise a selectable marker, e.g. a gene the product of which complements a defect in the host cell, such as the as the *dal* genes from *B.subtilis* or *B.licheniformis*, or one which confers antibiotic resistance such as ampicillin, kanamycin, chloramphenicol or tetracyclin resistance.

While intracellular expression may be advantageous in some respects, e.g. when using certain bacteria as host cells, it is generally preferred that the expression is extracellular. In order to obtain extracellular expression, the DNA construct and/or expression vector should normally further comprise a DNA sequence encoding a preregion, i.e. a signal peptide, permitting secretion of the expressed amylolytic enzyme, such as a *Pyrococcus* α-amylase or variant thereof, into the culture medium.

The procedures used to construct the DNA construct of the invention comprising ligating a DNA sequence encoding an amyolytic enzyme, e.g. the *Pyrococcus* α-amylase or variant thereof, the promoter, terminator and other elements, respectively, and to insert them into suitable vectors containing the information necessary for replication, are well known to persons skilled in the art (cf., for instance, Sambrook et al. (1989)).

The cell of the invention either comprising a DNA construct or an expression vector of the invention as defined above is advantageously used as a host cell in the recombinant production of a polypeptide of the invention. The cell may be transformed with the DNA construct of the invention, conveniently by integrating the DNA construct in the host chromosome, although the DNA construct may also exist as an extrachromosomal entity. However, the integration is generally considered to be an advantage as the DNA sequence is more likely to be stably maintained in the cell. Integration of the DNA constructs into the host chromosome may be performed according to conventional methods, e.g. by homologous recombination. Alternatively, the cell may be transformed with an expression vector as described below in connection with the different types of host cells.

The cell of the invention may be a cell of a higher organism such as a mammal or an insect, but is preferably a microbial cell, e.g. a bacterial or a fungal (including yeast) cell.

Examples of suitable bacteria are grampositive bacteria such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus thuringiensis* or *Streptomyces lividans* or *Streptomyces murinus,* or gramnegative bacteria such as *E.coli*. The transformation of the bacteria may for instance be effected by protoplast transformation or by using competent cells in a manner known *per se.*

The yeast organism may favourably be selected from a species of *Saccharomyces* or *Schizosaccharomyces,* e.g. *Saccharomyces cerevisiae.* The filamentous fungus may advantageously belong to a species of *Aspergillus,* e.g. *Aspergillus oryzae* or *Aspergillus niger.* Fungal cells may be transformed by a process involving protoplast formation and transformation of the protoplasts followed by regeneration of the cell wall in a manner known *per se.*

The recombinant *Bacillus* cell of the invention, which is different from a cell of *Bacillus licheniformis*, and which harbours a DNA construct comprising a DNA sequence encoding a *Pyrococcus* α-amylase or a variant thereof, is preferably selected from a strain of *Bacillus subtilis, Bacillus lentus, Bacillus brevis, Bacillus stearothermophilus, Bacillus alkalophilus, Bacillus amyloliquefaciens, Bacillus coagulans, Bacillus circulans, Bacillus lautus* or *Bacillus thuringiensis*.

The DNA construct harboured in the recombinant *Bacillus* cell of the invention is preferably a DNA construct as defined above, in which the DNA sequence encoding a *Pyrococcus* α-amylase or a variant thereof is derivable from a strain of *Pyrococcus woesei* or from a strain of *Pyrococcus furiosus*, in particular from the *Pyrococcus woesei* strain DSM 3773 or the *Pyrococcus furiosus* strain DSM 3638 or mutants or derivatives of said strains having retained their α-amylase producing capability.

In the process of the invention for producing an amylolytic enzyme, such as a *Pyrococcus* α-amylase or a variant thereof, a cell of the invention as defined above is cultured in a suitable culture medium under conditions permitting expression of the amylolytic enzyme, and the resulting enzyme is recovered from the culture.

The medium used to cultivate the cells may be any conventional medium suitable for growing the host cell in question. Suitable media are available from commercial suppliers or may be prepared according to published recipes (e.g. in catalogues of the American Type Culture Collection).

The amylolytic enzyme, e.g. the *Pyrococcus* α-amylase or variant thereof, may be recovered from the medium by conventional procedures including separating the cells from the medium by centrifugation or filtration, if necessary after disruption of the cells, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

The *Pyrococcus* α-amylase or variant thereof encoded by a DNA construct and/or produced by the process of the invention may be used in any process in which α-amylase activity is required, for instance in starch conversion or liquefaction processes used in the wine or fruit industry, for modifying starch to be used in the pulp and paper industry or for textile desizing using in the preparation of textiles. Also, the polypeptide may be used in baking in order to improve the properties of dough and/or baked products and for detergent purposes, e.g. as a constituent of a detergent additive or a detergent composition. Other uses involves degradation of biological waste, biomass conversion or degradation, or the preparation of energy from biological material.

Due to its high thermostability the *Pyrococcus* α-amylase or variant thereof of the invention is contemplated to be of particular use for processes in which a high thermostability is advantageous. An example of such a process is a starch liquefaction process, e.g. performed as disclosed in WO 90/11352, the contents of which is hereby incorporated by reference.

More specifically, the starch liquefaction process of the invention may be used for enzymatic conversion of starch into sugars, e.g. for the production of fuel alcohol or High Fructose Syrup (HFS). Suitable liquefaction conditions are up 120 minutes at 100-140°C, more preferred 1-60 minutes at 100-120°C, most preferred 1-30 minutes at 105-110°C, optionally followed by reduction of the temperature to be held in the range of 90-100°C for about 30-120 minutes. It is preferred not to add calcium salts to the aqueous starch slurry. The pH should be held within 3.5-6.0, more preferred 4.0-5.5, most preferred 4.2-4.8. A continuous process is preferred, and the heating is most preferably by jet-cooking. The dosage level of the α-amylase of the invention or a variant thereof is typically in the range of 5-500 NU, preferably 10-50 NU, per gram starch DS (dry substance). The starch concentration will usually be in the range 15-45% DS (w/w% dry substance), most often 25-35% DS.

The activity standard NU (which is an abbreviation of NOVO α-amylase unit) is the amount of enzyme which hydrolyses 5.26 mg of dissolved starch per hour at 37°C, pH 5.6 and 0.0043 M of Ca⁺⁺ over a 7-20 minute reaction time. A folder AF9/6 describing the analytical method is available on request to NOVO NORDISK A/S, DENMARK. The activity of the Pyrococcus α-amylase is determined at 60°C and related to a Termamyl standard, assayed under the same conditions.

The liquefied starch may hereafter be subjected to enzymatic saccharification in the presence of a glucoamylase, substantially without an intermediate pH adjustment. In this case the starch is liquefied with an α-amylase or variant of the invention at pH 3.5-6.0, more preferred at pH 4.0-4.5, most preferred pH 4.2-4.8. The liquefied starch may also be subjected to subsequent enzymatic saccharification in the presence of a glucoamylase in combination with a debranching enzyme such as pullulanase (see EP 63 909 for details) and/or an acid stable α-amylase from for example A. niger (see EP 140 410 for details).

The liquefaction process of the invention may also be used for producing ethanol. In this case the starch is liquefied with α-amylase at a pH of 3.5-6.0, more preferred 4.0-5.5, followed by saccharification with glucoamylase and simultaneous or subsequent fermentation with yeast. Thereafter the alcohol may be recovered by methods known in the art. Preferably the whole process is carried out at pH of about 4.5 without any intermediate pH adjustment, and simultaneous saccharification and fermentation is performed at 30-35°C for up to 96 hours. The liquefaction can be conducted either at low DS levels (15-20%) or high DS levels (20-40%). In the high DS processes, the DS level must be reduced to about 20% prior to fermentation to obtain about 10% alcohol by volume, which is about maximum that most yeast can tolerate.

The raw material for alcohol production may include refined starch such as wet milled corn starch; raw, unprocessed materials such as corn, wheat, rice, sorghum, cassawa and potato (whose starch content range from 15 to 80%); and other starch containing materials such as waste and by-products from industry.

Furthermore, the starch liquefaction may be performed by a process comprising
a) inserting a DNA construct of the invention encoding the *Pyrococcus* α-amylase or a variant thereof, optionally present in a suitable expression vector, into a suitable host organism,
b) culturing the host organism in a suitable culture medium under conditions permitting expression of the *Pyrococcus* α-amylase or variant thereof, and recovering the resulting *Pyrococcus* α-amylase or variant thereof from the culture, and
c) subjecting an aqueous starch slurry to enzymatic liquefaction in the presence of the *Pyrococcus* α-amylase or variant thereof recovered in step b), each of the steps being performed as described above.

### BRIEF DESCRIPTION OF THE DRAWINGS

The present invention is described in the following with reference to the appended drawings, in which
Fig. 1 illustrates the plasmid pSJ 1678, the nucleotide sequence of which is shown in SEQ ID No. 7,
Fig. 2 illustrates the plasmid pSJ 2467,
Fig. 3 illustrates the plasmid pSJ 2481,
Fig. 4 illustrates the plasmid pSJ 2482,
Figs. 5 and 6 illustrate the result of the southern analysis described in Example 5,
Fig. 7 illustrates the plasmids pSJ 2487 and pSJ 2488,
Fig. 8 illustrates the plasmids pSJ 2489 and pSJ 2490,
Fig. 9 illustrates the α-amylase activity of a DNA construct of the invention as further described in Example 6, and
Figs. 10 and 11 are chromatograms illustrating the distribution of oligosaccharides obtained from starch having been exposed to an α-amylase encoded by a DNA construct of the invention for 24 and 48 hours, respectively.

The following abbreviations are used on the plasmid drawings:
- rep: the gene for the pUB110 Rep protein (Gryczyn et al., 1978)
- cat: the gene for chloramphenicol acetyl transferase from pC194 (Horinouchi and Weisblum, 1982).
- p15A Ori: the replication functions from the *E. coli* cryptic plasmid p15A (Chang and Cohen, 1978)
- PamyM: the promoter region from the maltogenic amylase gene of *Bacillus stearothermophilus* (Diderichsen and Christiansen, 1988)
- kanD: the kanamycin resistance gene from pUB110, deleted for its own promoter
- pUB110 Ori: the double-stranded origin of replication for pUB110
- amyA.pfu: the alpha-amylase gene from *Pyrococcus furiosus*
- bla: the beta-lactamase gene from the pUC plasmids (Yanish-Perron et al., 1985)
- Plac: the beta-galactosidase promoter from the pUC plasmids
- 'amyA.pfu: the amyA.pfu gene truncated in the 5' end
- amyA.pfu': the amyA.pfu gene truncated in the 3' end

The present invention is further illustrated by the following examples which are not in any way intended to limit the scope of the invention as defined herein.

### MATERIALS AND METHODS

### Bacteria

*Pyrococcus furiosus* DSM 3638 and *Pyrococcus woesei* DSM 3773 available from the Deutsche Sammlung von Mikroorganismen und Zellkulturen GmbH, Brauschweig, Germany.

The strains were grown in medium according to the description as supplied from the DSM along with the strains.

*E*. *coli* SJ2 has been described by Diderichsen et al., 1990, and cells were prepared for and transformed by electroporation using a Gene Pulser™ electroporator from BIO-RAD as described by the supplier.

*B. subtilis* DN1885 has been described by Diderichsen et al., 1990, and competent cells were prepared and transformed as described by Yasbin et al., 1975.

### Plasmids

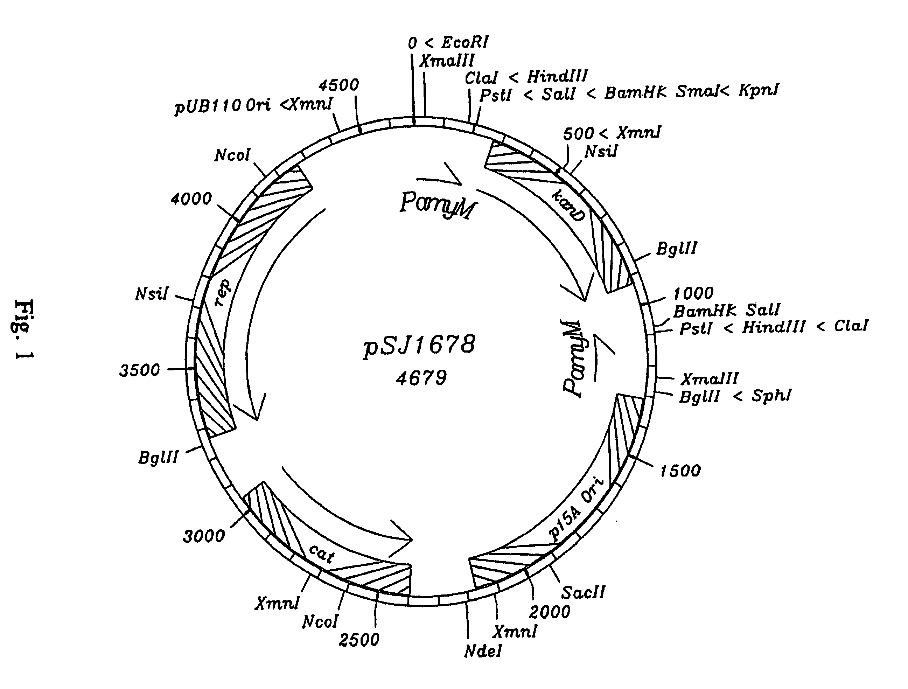

pSJ1678 (Fig. 1) was used as cloning vector in the construction of the gene library. The entire sequence of pSJ1678 is given in the appended sequence ID No. 7.

pUC19 (Yanish-Perron et al., 1985) was used for subclonings.

### General methods

The experimental techniques used to construct the plasmids were standard techniques within the field of recombinant DNA technology, cf. Sambrook et al., 1989.

Restriction endonucleases were purchased from New England Biolabs and Boehringer Mannheim and used as recommended by the manufacturers. T4 DNA ligase was purchased from New England Biolabs and used as recommended by the manufacturer.

Preparation of plasmid DNA from all strains was conducted by the method described by Kieser, 1984.

Amylase activity may be measured as absorption/ml at 620 nm using Phadebas tablets (Phadebas® Amylase Test; Pharmacia Diagnostics, SW). The assay is carried out for 15 min at 60°C, pH 7.3 in the presence of 0.15 nM calcium following the procedure described in the Novo Nordisk AF publication AF-207/-GB, avaliable upon request. The enzyme activity is compared to that of an enzyme standard and the result is expressed in the same unit as that of the enzyme standard (e.g. NU as defined herein).

### Media

Liquid cultures for plasmid preparation were grown in TY media supplemented with appropriate antibiotics

| | |
|---|---|
| Trypticase | 20 g/l |
| Yeast Extract | 5 g/l |
| FeCl₂.4H₂O | 6 mg/l |
| MnCl₂.4H₂O | 1 mg/l |
| MgSO₄.7H₂O | is mg/l |
| pH | 7.3 |

Liquid cultures for enzyme production and characterization were grown in Terrific Broth supplemented with relevant antibiotics

| | |
|---|---|
| Bacto-tryptone | 12 g |
| Bacto Yeast-extract | 24 g |
| Glycerol | 4 ml |
| Water to | 900 ml |

Following autoclaving, 100 ml of the following, separately autoclaved solution is added

| | |
|---|---|
| KH₂PO₄ | 0.17 H |
| K₂HPO₄ | 0.72 M |

Solid medium was LB agar

| | |
|---|---|
| Bacto-tryptone | 10 g/l |
| Bacto Yeast-extract | 5 g/l |
| NaCl | 10 g/l |
| Bacto agar | 15 g/l |
| Adjusted to pH 7.5 with NaOH | |

Media for visualization of amylase activity was LB agar containing, pr. 500 ml agar, 10 ml of a dyed amylopectin solution prepared as follows

12.5 g amylopectin (Serva 2000-4000 kD) is dissolved by boiling in 250 ml water, cooled to room temperature, 30 ml 4 M NaOH and 2.5 g cibacron Rot B are added, and the solution incubated overnight. pH is adjusted to 7 with 4 M HCL. 500 ml 96 % ethanol is added with stirring to precipitate the amylopectin as a red, viscous precipitate. The supernatant is discarded, the amylopectin dissolved in 200 ml water by slight heating, and the precipitation with ethanol repeated. The amylopectin is again dissolved in 200 ml water, autoclaved, and ready for use.

### EXAMPLES

### EXAMPLE 1

### Cloning of the Pyrococcus furiosus α-amylase gene

Genomic DNA from *Pyrococcus furiosus* DSM3638 was isolated by the method of Pitcher et al., 1989. Approximately 100 µg DNA was partially digested with Sau3A, size fractionated on a sucrose gradient, and fragments between 3 and 7 kb were pooled.

The cloning vector pSJ1678 was digested with BamHI, and a 3.8 kb fragment was purified from an agarose gel. Approximately 0.75 µg vector fragment was ligated to appr. 4 µg size-fractionated *P. furiosus* chromosomal DNA, and used to transform *E. coli* SJ2 by electroporation.

The gene bank was plated on LB plates containing dyed amylopectin and supplemented with 10 µg/ml chloramphenicol. Following overnight incubation at 37°C, each plate was replica plated onto two new plates which were then incubated overnight at 37°C. One of these was subsequently incubated at 60°C overnight.

Clear halos indicating degradation of the amylopectin was observed around 5 colonies (among a total of 10000) on the 60°C plates, whereas no halos were observed around the colonies on the plates that were kept at 37°C. These 5 strains taken from the 37°C plates were kept as SJ2463-SJ2467.

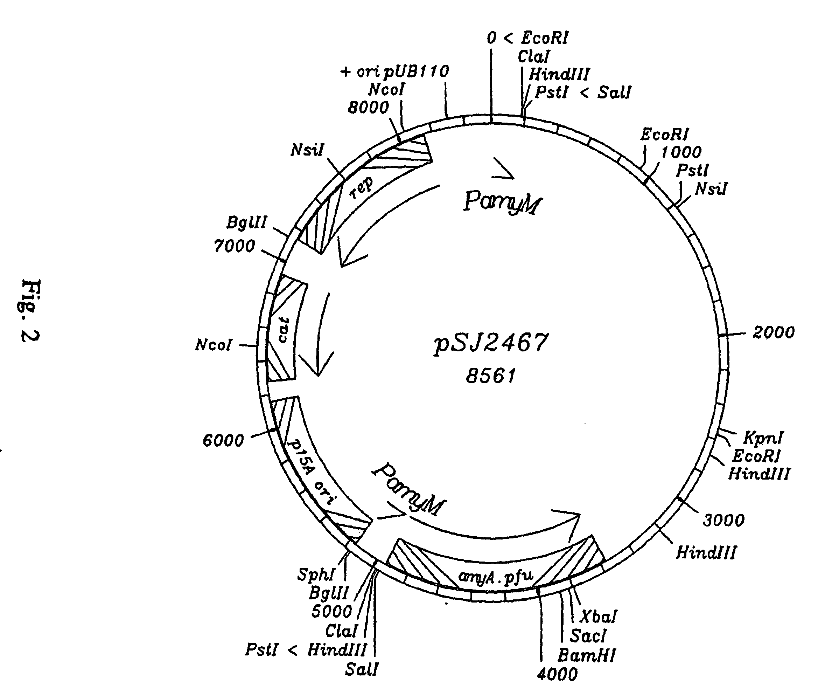

Restriction digests revealed that the *P. furiosus* DNA insert on the four clones SJ2463, SJ2464, SJ2465 and SJ2467 shared a common DNA region without the inserts being totally identical, whereas the DNA contained on pSJ2466 appeared unrelated to these four clones. pSJ2467 (Fig. 2) contained an insert of approximately 4.5 kb and was used for further analysis.

### EXAMPLE 2

### Starch degradation using amylase produced from SJ2467

*E. coli* SJ 2467 (the above described *E. coli* SJ 2 harbouring pSJ2467) was grown in Terrific broth medium supplemented with 6 µg/ml chloramphenicol for 3 days at 37°C. The *E. coli* cells in the culture broth were lysed by sonication.

One sample was directly sterile filtered, another sample sterile filtered after a brief heat-treatment.

2 g waxy corn starch were slurried with 10 ml 0.1 M acetate buffer, with pH values of 4.3, 5.0 and 5.5, containing 40 ppm Ca⁺⁺, in 180 x 18 mm glass culture tubes. 2 ml heat-treated (105°C, 5 min.) *E*. *coli* sonicated extract, containing approximately 2 NU/ml *Pyrococcus furiosus* amylase were added and the pH controlled at ambient temperature.

The tightly sealed glass tubes were transferred to a thermostated oil-bath at 105°C and vigorously agitated. Within minutes of gelatinization, the starch was liquified. Samples were taken periodically and tested with iodine (1 drop 0.1 M iodine in 5 ml deionized water). The following results were obtained.

| Sample | Time (hours) | pH | Iod. colour |
|---|---|---|---|
| pH 4.6 | 0.5 | - | purple |
| | 6 | - | red |
| | 24 | 4.5 | brown |
| pH 5.3 | 0.5 | - | purple |
| | 6 | - | red |
| | 24 | 5.1 | brown |
| pH 5.5 | 0.5 | - | purple |
| | 6 | - | red |
| | 24 | 5.4 | brown |

This test demonstrates that *P. furiosus* amylase, expressed in *E*. *coli*, is active and stable at high temperature and low pH, in the presence of starch.

Furthermore, the test demonstrates that it is possible to obtain expression of the extremely thermophilic *P. furiosus* amylase in active form in *E. coli*. It may be considered surprising that the *Pyrococcus* α-amylase, which in it's native environment is synthesized, secreted and folded into an active three-dimensional structure at 100°C, also may be produced in active form at 37°C.

In a further experiment 2 g waxy corn starch were slurried with 2 ml 1 M acetate buffer, pH 5.5, in a 180 x 18 mm glass culture tube. 10 ml *E. coli* sonicated extract, containing approximately 1 NU/ml *P*. *furiosus* amylase were added and the pH adjusted to 5.5 at ambient temperature.

The tightly sealed glass tube was transferred to a thermostated oil-bath at 105°C and vigorously agitated. Within seconds of gelatinization, the starch was liquefied. Samples were taken periodically and tested with iodine (1 drop 0.1 M iodine in 5 ml deionised water). Samples were also taken for HPLC analysis. The following results were obtained.

| Time (hours) | pH | Iod. colour |
|---|---|---|
| 1 | - | red |
| 2 | - | brown/red |
| 24 | 5.2 | yellow |
| 48 | 4.8 | yellow |

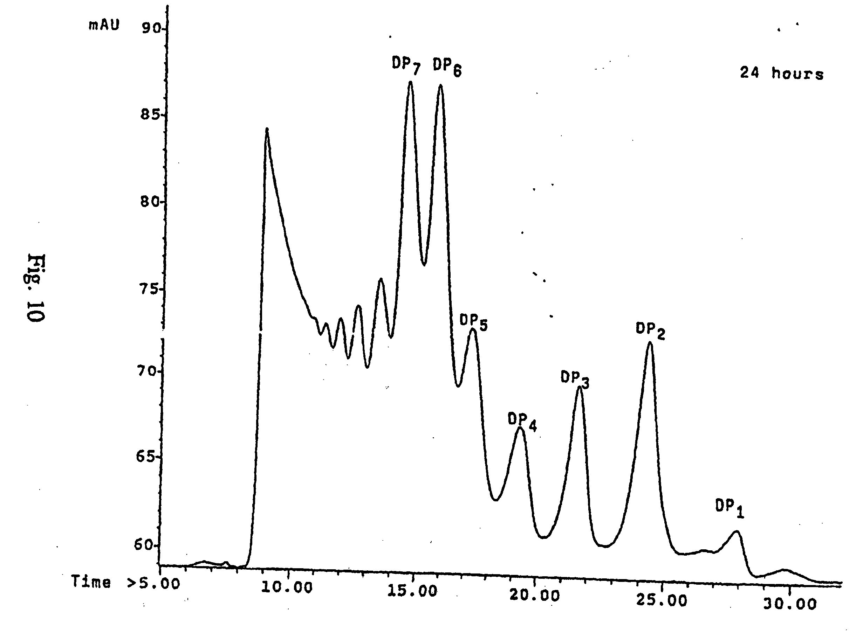

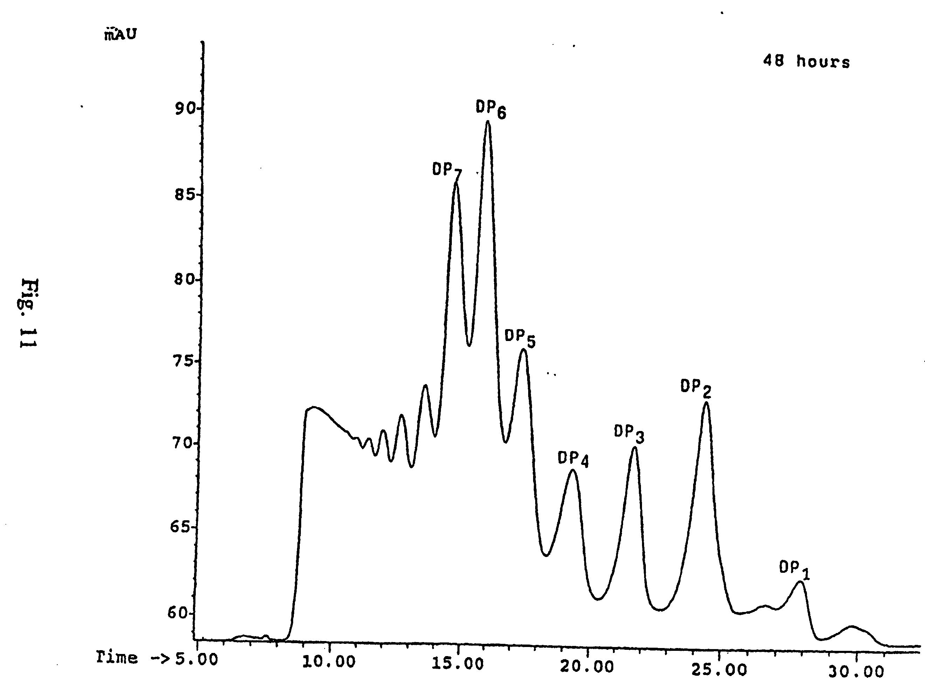

The distribution of oligosaccharides seen in the chromatograms shown in Figs. 10 and 11 is typical of endo-amylase attack. The major oligosaccharides formed on prolonged hydrolysis are DP5, DP6 and DP7 (where DP=degree of polymerization).

### EXAMPLE 3

### Subcloning of P. furiosus α-amylase gene

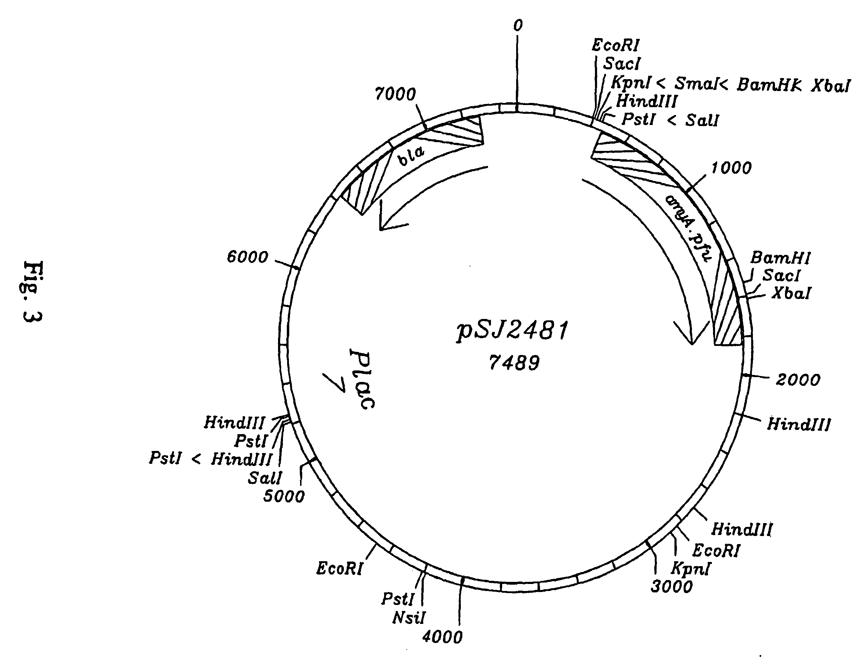

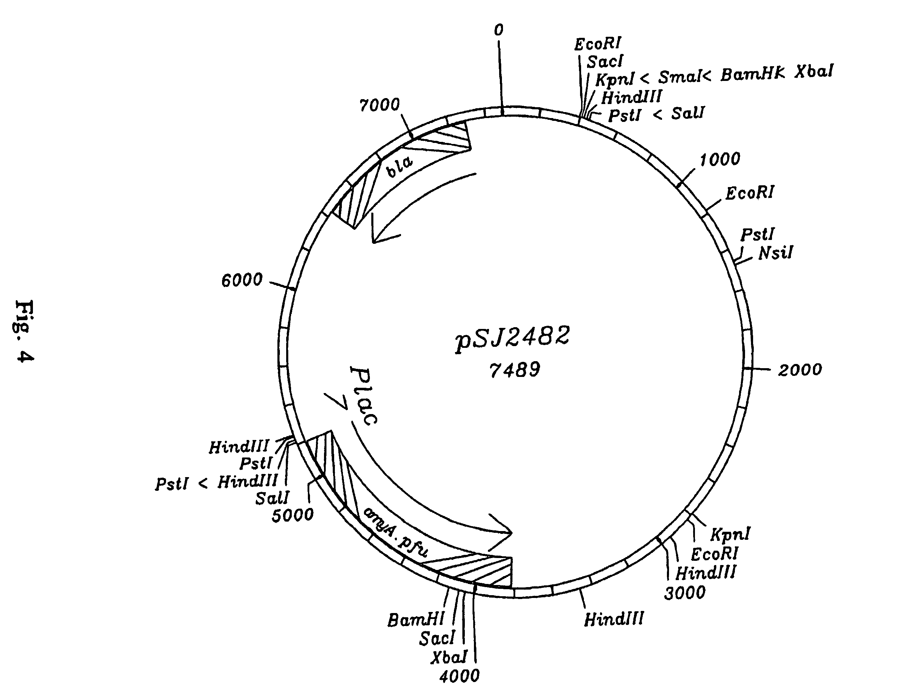

pS2467 was digested with ClaI, and the 4.5 kb fragment containing the α-amylase gene was ligated to AccI digested pUC19 DNA and the ligation mixture transformed into *E*. *coli* SJ2. Transformants were obtained containing the insert in each of the two possible orientations with respect to the cloning vectors. These were SJ2481 containing pSJ2481 (Fig. 3), and SJ2482 containing pSJ2482 (Fig. 4).

Both clones produce α-amylase as visualized by the appearance of clear halos on dyed amylopectin plates after incubation at 60°C. The amylase-producing transformants appear somewhat sick as compared to transformants containing the pUC19 vector plasmid only. They form smaller, more translucent colonies.

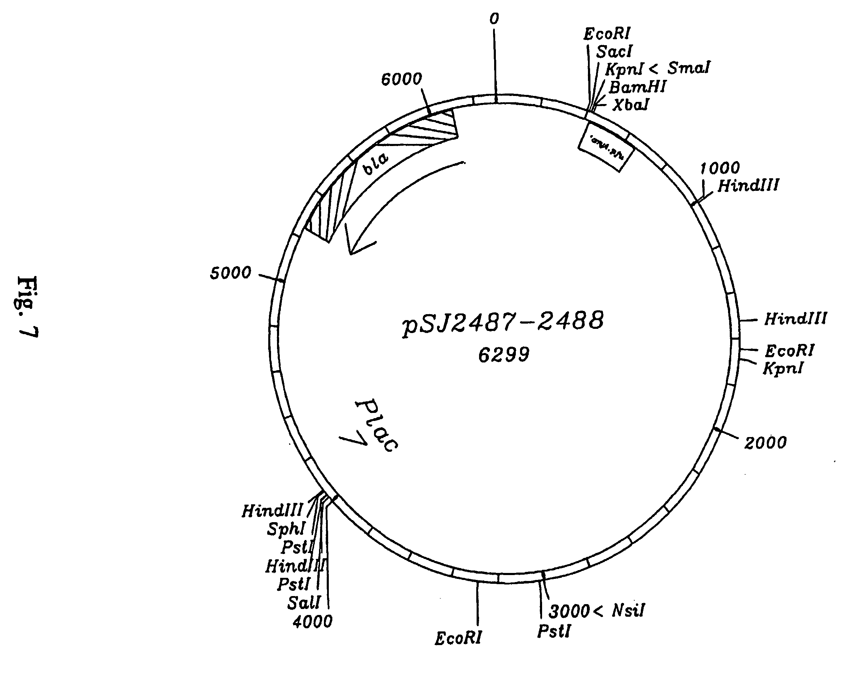

Further subclonings were performed from pSJ2481. pSJ2487 (Fig. 7) was constructed by deletion of the 1 kb XbaI fragment from pSJ2481 and transformation of the religated plasmid into *E*. *coli* SJ2. The resulting transformants were not able to produce halos on LB plates containing dyed amylopectin, indicating that this deletion had removed a DNA region of importance for expression of an active amylase protein.

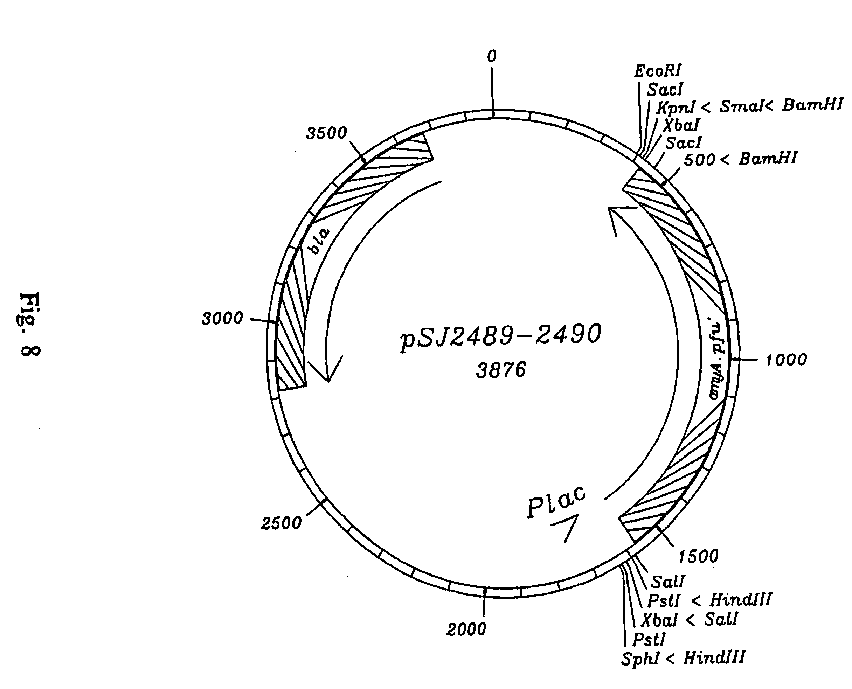

The 1 kb XbaI fragment from pSJ2481 was inserted into XbaI digested pUC19, to give pSJ2489 and pSJ2490 (identical. Fig. 8).

### EXAMPLE 4

### DNA sequences

The ends of the insert on several subclones in pUC19 were sequenced directly on the double-stranded plasmids using Sequenase™ and oligonucleotide primers hybridizing just outside the pUC19 multilinker region.

The resulting sequences are given in SEQ ID Nos. 2, 3, 4, 5 and 6.

The SEQ ID No. ID 2 is derived from pSJ2490, and read from the end of the insert next to the EcoRI site in the pUC polylinker.

The SEQ ID No. 3 is derived from pSJ2489, and read from the end of the insert next to the HindIII site in the pUC polylinker.

The SEQ ID No. 4 is derived from pSJ2487, and read from the end of the insert next to the EcoRI site in the pUC polylinker.

The SEQ ID No. 5 is derived from pSJ2482, and read from the end of the insert next to the EcoRI site in the polylinker.

The SEQ ID No. 6 is derived from pSJ2482, and read from the end of the insert next to the HindIII site in the pUC polylinker.

Based on the DNA sequences given herein, it will be possible to synthesize oligonucleotide primers that can be used to amplify from chromosomal DNA of Pyrococcus furiosus in a PCR reaction a DNA fragment identical to the entire Pyrococcus furiosus DNA insert contained on pSJ2467 and pSJ2481/2482, thereby reconstructing these plasmids from available material.

### EXAMPLE 5

### Southern analysis

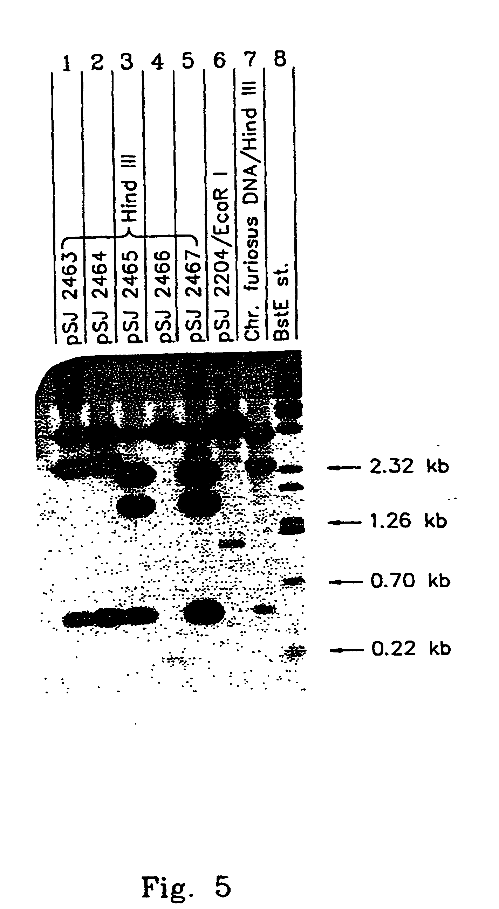

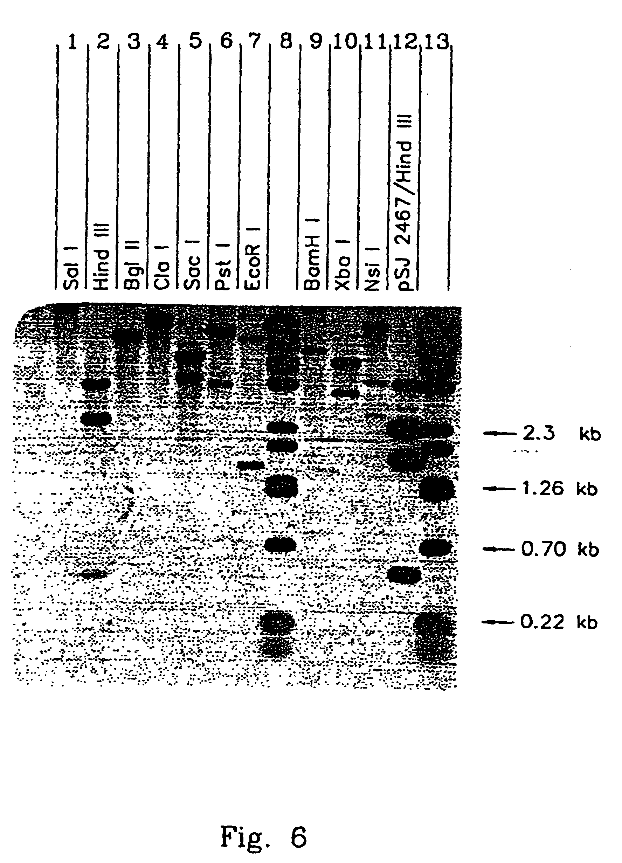

pSJ2481 was ³²P-labelled by nick-translation using a commercial kit obtained from Amersham, and used as probe in a southern analysis. Hybridization was overnight at 60°C in 10x Denhardts solution, 1%SDS, 10 mM EDTA and 5x SSC, followed by two 15 min. washes in 2x SSC, 0.1% SDS at room temperature and one 15 min. wash at 60°C. The resulting exposures are shown in Fig. 5 and Fig. 6.

Fig. 5 reveals
1) that pSJ2463, pSJ2464, pSJ2465 and pSJ2467 contains a common DNA region as previously stated (a HindIII fragment of approx. 0.5 kb is common to pSJ2463 (lane 1), pSJ2464 (lane 2), pSJ2465 (lane 3), pSJ2467 (lane5) and to chromosomal *P. furiosus* DNA (lane 7)).
2) that the insert on pSJ2481 is derived from the chromosome of *P. furiosus.* (lane 7). Fig. 6 reveals
3) that a homologous DNA region exist in the chromosome of *P*. *woesei* (the chromosomal *P. woesei* DNA being isolated by the method according to Pitcher et al. 1989). Thus pSJ2481 hybridizes to exactly the same fragments in HindIII digested *P*. *woesei* DNA (lane 2) as in HindIII digested *P. furiosus* DNA (Fig. 5, lane 7). For clarity, a longer exposure of lane 2 is added as lane 0 of figure 6 to reveal the 0.5 kb HindIII fragment.

pSJ2481, or the sequence information given herein obtained from pSJ2481, can therefore be used as a tool to identify and thereby assist cloning of the α-amylase gene from the chromosome of either *P. furiosus* or *P. woesei*.

### EXAMPLE 6

### Expression of the α-amylase gene in Bacillus subtilis

The plasmid pSJ1678 used for construction of the gene library is a shuttle vector able to replicate in both *E. coli* and *B. subtilis*.

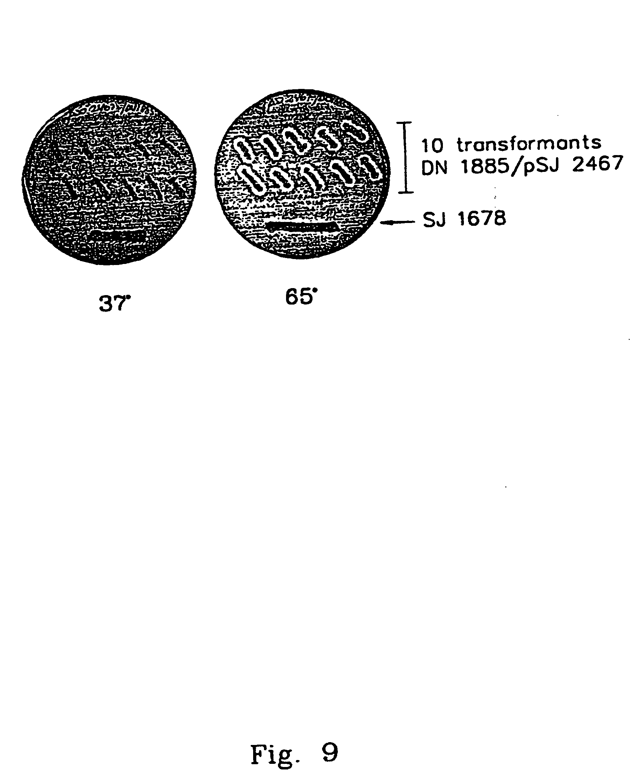

To test for expression of the amylase activity in *B. subtilis*, pSJ2467 was therefore transformed into competent cells of DN1885 selecting for resistance to chloramphenicol (6 µg/ml) on LB plates containing dyed amylopectin. 10 transformants were picked onto two new plates with dyed amylopectin, along with SJ1678 which is DN1885/pSJ1678 as a control. After incubation overnight at 37°C one plate was transferred to 65°C, whereas the other was kept at 37°C. 7 hours later was degradation of the amylopectin around the 10 transformants with pSJ2467 apparent on the plate incubated at 65°C as formation of a clear halo. No halo was formed around the control strain (Fig. 9).

The fact that expression of amylase activity from a *Pyrococcus* α-amylase gene can be obtained in *Bacillus subtilis* without any modification of the gene, e.g. in form of modification or replacement of the ribosome binding site to allow more efficient initiation of translation, is surprising indeed. Generally, the majority of cloned genes from non-gram positive organisms fail to express from their own expression signals in *B*. *subtilis* (Mountain, A., 1989), and their has to our knowledge been no prior reports of direct expression of a gene from *Pyrococcus* in *B. subtilis*.

### EXAMPLE 7

4.5 kb of the *P. furiosus* DNA insert cloned on pSJ2467 (Example 1) was sequenced on both strands, using Sequenase™ and a combination of subclones and oligonucleotide primers based on previously determined sequences.

The open reading frame corresponding to the α-amylase gene was localized by subcloning (the ability of individual subclones to produce α-amylase was assayed on plates containing dyed amylopectin).

The α-amylase encoded by this open reading frame revealed homology to α-amylases and other starch-degrading enzymes from a variety of organisms including bacteria, insects and plants. When aligned with a *B. licheniformis* α-amylase a degree of identity of about 36% could be observed when 18 gaps were introduced at various sites in the sequence.

The DNA sequence of the α-amylase coding region, including the signal peptide coding region, is shown in Seq. ID No. 1. On the basis of the DNA sequence shown in SEQ ID No. 1 the amino acid sequence of the signal peptide (Seq. ID 8) and of the mature α-amylase (Seq. ID 9) have been deduced.

On the map of pSJ2467 (fig. 2), the α-amylase gene is located between position 4.5 and 3.0, reading counterclockwise.

### REFERENCES CITED IN THE SPECIFICATION

S.L. Beaucage and M.H. Caruthers, Tetrahedron Letters 22, 1981, pp. 1859-1869, or the method described by Matthes et al., The EMBO J. 3, 1984, pp. 801-805.
Brown et al., Applied and Environmental Microbiology, July 1990, pp. 1985-1991
Chang, A. C. Y., Cohen, S. N. (1978). Construction and Amplification of Amplifiable Multicopy DNA Cloning Vehicles Derived from the p15A Cryptic Miniplasmid. J. Bacteriol., 134, 1141-1156.
Diderichsen, B., Wedsted, U., Hedegaard, L., Jensen, B. R., Sjøholm, C. (1990). Cloning of *aldB*, which encodes α-acetolactate decarboxylase, an exoenzyme from *Bacillus brevis*. J. Bacteriol., 172, 4315-4321.
Diderichsen, B. and Christiansen, L. Cloning of a maltogenic α-amylase from Bacillus stearothermophilus, FEMS Microbiol. Lett. 5653-60, 1988.
Gryczan, T. et al. (1978). Characterization of Staphylococcus aureus plasmids introduced by transformation into Bacillus subtilis. J. Bacteriol., 134, 318-329.
Horinouchi et al. (1982b) "Nucleotide sequence and functional map of pE194, a plasmid that specifies inducible resistance to macrolide, lincosamide, and streptogramin type B antibiotics" J. Bacteriol., 150, 804-814.
Hudson et al., 1989, Practical Immunology, Third edition (1989), Blackwell Scientific Publications,
Kieser. T. (1984). Factors affecting the isolation of CCC DNA from *Streptomyces lividans* and *Escherichia coli*. Plasmid 12, 19-36.
Koch et al., FEMS Microbiology Letters 71 (1990) 21-26
Koch et al., Arch. Microbiol. 155 (6), 1991, pp. 572-578
Lipman and Pearson (1985), Science 227, 1435 (1985)
Matthes et al., EMBO J. 3, 1984, pp. 801-805
Mountain, A. (1989). In "Biotechnology Handbooks Vol. 2. Bacillus" Ed. Harwood, C. R. Plenum Press, New York, 1989, pp 73-114.
Pitcher, D. G., Saunders, N. A., Owen, R. J. (1989). Rapid extraction of bacterial genomic DNA with guanidium thiocyanate. Lett. Appl. Microbiol., 8, 151-156.
Podkovyrov, Journ. of Bacteriol., 1992, Vol. 174, pp. 5400-5405.
R.K. Saiki et al., Science 239, 1988, pp. 487-491.
Sambrook et al., Molecular Cloning A laboratory manual, 2nd Ed., Cold Spring Harbor, 1989.
Svensson, B., FEBS Letters, 1988, Vol. 230, pp. 72-76.
Yanish-Perron, C., Vieira, J., Messing, J. (1985). Improved M13 phage cloning vectors and host strains Nucleotide sequences of the M13 mp18 and pUC19 vectors. Gene 33, 103-119.
Yasbin, R. E., Wilson, G. A., Young, F. E. (1975). Transformation and transfection in lysogenic strains of *Bacillus subtilis* Evidence for selective induction of prophage in competent cells. J. Bacteriol., 121, 296-304.
Zhou, FEBS Letters, 1989, Vol. 255, pp. 37-41.

## Claims

1. A DNA construct comprising a DNA sequence encoding a *Pyrococcus* alpha-amylase shown in SEQ ID NO: 1 or a DNA sequence encoding an alpha-amylase being at least 70% identical with the DNA sequence shown in SEQ ID NO: 1.

2. A DNA construct of claim 1, in which the DNA sequence is derivable from a thermophilic archaebacterium.

3. The DNA construct of claim 2, in which the DNA sequence is derivable from a strain of *Pyrococcus woesei* or from a strain of *Pyrococcus furiosus*.

4. The DNA construct of claim 3, in which the DNA sequence is derivable from the *Pyrococcus woesei* strain DSM 3773 or the *Pyrococcus furiosus* strain DSM 3638, or from a mutant or derivative of any of these strains having an alpha-amylase producing capability.

5. A vector harbouring a DNA construct of any of claims 1-4.

6. The vector of claim 5, which is a plasmid or a bacteriophage.

7. The vector of claim 5 or 6, which is an expression vector further comprising DNA sequences permitting expression of the amylolytic enzyme, such as a *Pyrococcus* alpha-amylase.

8. A host cell harbouring a DNA construct of any of claims 1-4 or a vector of any of claims 5-7.

9. The host cell of claim 8, which is a microorganism.

10. The host cell of claim 9, which is a bacterium or a fungus.

11. The host cell of claim 10, which is a grampositive bacterium such as *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus thuringiensis* or *Streptomyces lividans* or *Streptomyces murinus,* or gramnegative bacteria such as *E.coli*.

12. A process for producing a *Pyrococcus* alpha-amylase, comprising culturing a cell of claims 8-11 in a suitable culture medium under conditions permitting expression of the amylolytic enzyme, and recovering the resulting amylolytic enzyme from the culture.

## Patentansprüche

1. DNA-Konstrukt, umfassend eine DNA-Sequenz, die eine in SEQ ID NR. 1 dargestellte alpha-Amylase von *Pyrococcus* kodiert, oder eine DNA-Sequenz, die eine alpha-Amylase, die mindestens zu 70% mit der in SEQ ID NR. 1 dargestellten DNA-Sequenz identisch ist, kodiert.

2. DNA-Konstrukt nach Anspruch 1, in welchem die DNA-Sequenz von einem thermophilen Archaebakterium ableitbar ist.

3. , DNA-Konstrukt nach Anspruch 2, in welchem die DNA-Sequenz von einem Stamm von *Pyrococcus woesei* oder einem Stamm von *Pyrococcus furiosus* ableitbar ist.

4. DNA-Konstrukt nach Anspruch 3, in welchem die DNA-Sequenz von dem Stamm DSM 3773 von *Pyrococcus woesei* oder dem Stamm DSM 3638 von *Pyrococcus furiosus* oder einem Mutanten oder Derivat von einem dieser Stämme mit alpha-Amylase-erzeugender Fähigkeit ableitbar ist.

5. Vektor, der ein DNA-Konstrukt nach einem der Ansprüche 1-4 beinhaltet.

6. Vektor nach Anspruch 5, der ein Plasmid oder ein Bakteriophage ist.

7. Vektor nach Anspruch 5 oder 6, der ein Expressionsvektor ist, ferner umfassend DNA-Sequenzen, die die Expression des amylolytischen Enzyms wie einer alpha-Amylase von *Pyrococcus* gewähren.

8. Wirtszelle, die ein DNA-Konstrukt nach einem der Ansprüche 1 - 4 oder einen Vektor nach einem der Ansprüche 5 - 7 beinhaltet.

9. Wirtszelle nach Anspruch 8, die ein Mikroorganismus ist.

10. Wirtszelle nach Anspruch 9, die ein Bakterium oder ein Pilz ist.

11. Wirtszelle nach Anspruch 10, die ein grampositives Bakterium wie *Bacillus subtilis, Bacillus licheniformis, Bacillus lentus, Bacillus brevis, Bacillus stearothermophilus, Bacillus alkalophilus*, *Bacillus amyloliquefaciens, Bacillus coagulans, Bacillus circulans, Bacillus lautus, Bacillus thuringiensis* oder *Streptomyces lividans* oder *Streptomyces murinus* oder ein gramnegatives Bakterium wie *E. coli* ist.

12. Verfahren zur Herstellung einer alpha-Amylase von *Pyrococcus*, umfassend das Züchten einer Zelle nach den Ansprüchen 8 - 11 in einem geeigneten Kulturmedium unter Bedingungen, die die Expression des amylolytischen Enzyms gewähren, und Gewinnen des erhaltenen amylolytischen Enzyms aus der Kultur.

## Revendications

1. Structure d'ADN comportant une séquence d'ADN codant pour une alpha-amylase de *Pyrococcus* représentée dans la SEQ ID N° 1, ou une séquence d'ADN codant pour une alpha-amylase qui est au moins identique à 70 % avec la séquence d'ADN représentée dans la SEQ ID N° 1.

2. Structure d'ADN selon la revendication 1, dans laquelle la séquence d'ADN peut être dérivée d'une archéobactérie thermophile.

3. Structure d'ADN selon la revendication 2, dans laquelle la séquence d'ADN peut être dérivée d'une souche de *Pyrococcus woesei* ou d'une souche de *Pyrococcus furiosus*.

4. Structure d'ADN selon la revendication 3, dans laquelle la séquence d'ADN peut être dérivée de la souche DSM 3773 de *Pyrococcus woesei* ou de la souche DSM 3638 de *Pyrococcus furiosus,* ou d'un mutant ou dérivé de l'une quelconque de ces souches ayant une capacité de production d'alpha-amylase.

5. Vecteur présentant une structure d'ADN selon l'une quelconque des revendications 1 à 4.

6. Vecteur selon la revendication 5, qui est plasmide ou un bactériophage.

7. Vecteur selon la revendication 5 ou 6, qui est un vecteur d'expression comportant de plus des séquences d'ADN permettant l'expression de l'enzyme amylolytique, telle que l'alpha-amylase de *Pyrococcus.*

8. Cellule hôte présentant une structure d'ADN selon l'une quelconque des revendications 1 à 4 ou un vecteur selon l'une quelconque des revendications 5 à 7.

9. Cellule hôte selon la revendication 8, qui est un micro-organisme.

10. Cellule hôte selon la revendication 9, qui est une bactérie ou un champignon.

11. Cellule hôte selon la revendication 10, qui est une bactérie à gram positif telle que *Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, Bacillus lautus, Bacillus thuringiensis* ou *Streptomyces lividans* ou *Streptomyces murinus,* ou des bactéries à gram négatif telles que *E. coli.*

12. Procédé pour produire une alpha-amylase de *Pyrococcus,* comportant la culture d'une cellule selon les revendications 8 à 11, dans un milieu de culture adapté sous des conditions permettant l'expression de l'enzyme amylolytique, et la récupération de l'enzyme amylolytique résultante à partir de la culture.


### SEQUENCE LISTING

(1) GENERAL INFORMATION
   (i) APPLICANT
      (A) NAME NOVO NORDISK A/S
      (B) STREET Novo Alle
      (C) CITY Bagsvaerd
      (E) COUNTRY DENMARK
      (F) POSTAL CODE (ZIP) DK-2880
      (G) TELEPHONE +45 44448888
      (H) TELEFAX +45 4449 3256
      (I) TELEX 37304
   (ii) TTTLE OF INVENTION An amylolytic enzyme
   (iii) NUMBER OF SEQUENCES 52
   (iv) COMPUTER READABLE FORM
      (A) MEDIUM TYPE Floppy disk
      (B) COMPUTER IBM PC compatible
      (C) OPERATING SYSTEM PC-DOS/MS-DOS
      (D) SOFTWARE PatentIn Release #1.0, Version #1.25 (EPO) Novo Nordisk A/S
(2) INFORMATION FOR SEQ ID NO 1
   (i) SEQUENCE CHARACTERISTICS
      (A) LENGTH 1380 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 1 
GTGAACATAA AGAAATTAAC ACCCCTCCTA ACTCTATTAC TGTTTTTTAT AGTACTAGCA 60
AGTCCAGTAA GTGCAGCAAA ATACTTGGAG CTTGAAGAGG GAGGAGTTAT AATGCAAGCA 120
TTCTATTGGG ATGTTCCAGG GGGAGGAATT TGGTGGGATC ATATAAGATC GAAGATTCCT 180
GAATGGTATG AAGCTGGAAT CTCTGCAATA TGGCTACCTC CACCAAGCAA GGGGATGAGT 240
GGAGGATATT CAATGGGCTA CGATCCCTAT GATTACTTTG ATCTCGGCGA GTACTACCAG 300
AAGGGAACTG TAGAGACGCG TTTTGGATCA AAAGAAGAAC TAGTGAGATT GATACAAACT 360
GCCCATGCCT ATGGAATAAA GGTAATCGCC GATGTAGTTA TAAACCACAG GGCTGGTGGT 420
GACCTAGAAT GGAACCCCTT CGTTGGAGAT TACACATGGA CAGACTTTTC TAAAGTTGCC 480
TCAGGGAAAT ATACAGCTAA CTATCTGGAC TTCCATCCAA ACGAGCTTCA TTGTTGTGAC 540
GAAGGAACCT TTGGAGGATT TCCAGATATA TGTCATCACA AAGAGTGGGA TCAGTACTGG 600
CTATGGAAGA GCAATGAGAG TTATGCTGCT TATTTAAGAA GCATAGGATT TGATGGTTGG 660
AGATTTGACT ATGTTAAGGG CTATGGAGCT TGGGTTGTCA GAGACTGGCT TAATTGGTGG 720
GGAGGTTGGG CAGTTGGAGA GTACTGGGAC ACAAATGTAG ATGCACTACT AAGCTGGGCA 780
TATGAGAGTG GTGCAAAGGT CTTTGACTTC CCGCTCTACT ATAAAATGGA TGAAGCATTT 840
GACAATAACA ACATTCCAGC ATTAGTCTAT GCCCTACAAA ACGGACAAAC TGTAGTTTCG 900
 
AGAGATOCAT TTAAGGCAGT AACTTTOGTT GOCAATCATG ACACAGATAT AATATGGAAC 960
AAGTATOCAG CATATGOGTT CATATTGACA TATGAGGGAC AGOCAGTAAT ATTCTACAGG 1020
GACTTTGAGG AATGGCTGAA CAAGGATAAG CTAATTAAOC TCATTTGGAT OCATGATCAT 1080
TTGGCAGGAG GAAGCACAAC AATTGTCTAC TACGACAAOG ATGAGCTCAT ATTTGTGAGA 1140
AATGGAGATT CTAGAAGGOC TGGGCTTATA ACTTACATTA ACTTGAGOOC TAACTGGGTT 1200
GGTAGGTGGG TATAOGTTOC AAAGTTTGCA GGGGCTTGTA TTCATGAATA CACTGGAAAC 1260
CTAGGAGGAT GGGTAGATAA AAGAGTAGAT AGTAGOGGAT GGGTATAOCT AGAGGCAOCA 1320
OCTCAOGATC CAGCTAAOGG CTACTATGGG TACTOOGTAT GGAGTTATTG TGGTGTTGGG 1380
(2) INFORMATION FOR SEQ ID NO 2
   (i) SEQUENCE CHARACTERISTICS
      (A) LENGTH 303 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 2 
TCTAGAATCT CCATTTCTCA CAAATATCAG CTCATCGTTG TCGTAGTAGA CAATTGTTGT 60
GCTTCCTCCT GCCAAATGAC TATGGATCCA AATGAGGTTA ATTAGCTTAT CCTTGTTCAG 120
CCATTCCTCA AAGTCCCTGT AGAATATTAC TGGCTGTCCC TCATATGTCA ATATGAACGC 180
ATATGCTGGA TACTTGTTCC ATATTATATC TGTGTCATGA TTGGCAACCA AAGTACTGCC 240
TTAAATGGAT CTCTCGAAAC TACAGTTTGT AACGTTTCTA GGGCATAGAC TAATTAGCTG 300
GAA 303
(2) INFORMATION FOR SEQ ID NO 3
   (i) SEQUENCE CHARACTERISTICS
      (A) LENGTH 117 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 3 
GATCACGTGA ACATAAAGAA ATTAAGACCC CTGCTAACTC TATTACTGTT TTTTATAGTA 60
CTAGCAAGTC CAGTAGTGCA GCAAAATACT TGGAGCTTGA ACAGGCANGA GTTATAA 117
(2) INFORMATION FOR SEQ ID NO 4
   (i) SEQUENCE CHARACTERISTICS
      (A) LENGTH 207 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 4 
TCTAGAAGGC CTGGGCTTAT AACTTACATT AACTTGAGCC CTAACTGGGT TGGTAGGTGG 60
GTATACTTCC AAAGTTTGCA GGGGCTTGTA TCATGAATAC ACGGAAACCT AGGAGGATGG 120
GAGATAAAAG AGTAGATAGT AGCGGATGGG TATACCTAGA GGCACCACCT CACGATCCAG 180
CTAACGGCTA CTATCGGTAC TCCGTAT 207
(2) INFORMATION FOR SEQ ID NO 5
   (i) SEQUENCE CHARACTERISTICS
      (A) LENGTH 237 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 5 
GATCCAAAGT GTTATCTGGA AATGGGTAGA ACAATACGTC TGAAGAAATT GGGACATCTT 60
TTGTTATATC AGTATGGGTA CTTGATTACG AAAATAAAAA GCTCTACAGA GGATTCACTA 120
TAGTGAATTA TGAAATCAAG GACATGAGAA AGGGGTTCAA AAAAATAGTT AAGGTAAATA 180
TTCACAAACT ACCCTCCAGC GAACTTGGAT CTAATAGAAC AGCATCCATT TCAAGGG 237
(2) INFORMATION FOR SEQ ID NO 6
   (i) SEQUENCE CHARACTERISTICS
      (A) LENGTH 192 base pairs
      (B) TYPE nucleic acid
      (C) STRANDEDNESS single
      (D) TOPOLOGY linear
   (ii) MOLECULE TYPE DNA (genomic)
   (vi) ORIGINAL SOURCE
      (A) ORGANISM Pyrococcus furiosus
      (B) STRAIN DSM 3638
   (xi) SEQUENCE DESCRIPTION SEQ ID NO 6 
GATCACGTGA ACATAAAGAA ATTAACNCCC CTCCTAACTC TATTACTGTT TTTTATAGTA 60
CTAGCAAGTC CAGTAAGTGC AGCAAAATAC TTGGAGCTTG AAGAGGGAGG AGTTATAATG 120
CAAGCATTCT ATTGGGATGT TCCAGGNGGA GGATTTGGTG GGATCATATA AGATCGAAGA 180
TTCCTGAATG GG 192
(2) INFORMATION FOR SEQ ID NO 7
   (i) SEQUENCE CHARACTERISTICS
      (A) LENGTH 4679 base pairs
      (B) TYPE nucleic acid
      (C) STRANDEDNESS single
      (D) TOPOLOGY linear
   (ii) MOLECULE TYPE DNA (genomic)
   (xi) SEQUENCE DESCRIPTION SEQ ID NO 7 
GAATTCCGGC CCAACGATGG CTGATTTCCG GGTTGACGGC CGGCGGAACC AAGGGGTGAT 60
CGGTCGGCGG AAATGAAGGC CTGCGGCGAG TGCGGGCCTT CTGTTTTGAG GATTATAATC 120
AGAGTATATT GAAAGTTTCG CGATCTTTTC GTATAATTGT TTTAGGCATA GTGCAATCGA 180
TAAGCTTGGC TGCAGGTCGA CGGATCCCCG GGTACCCATT CTTATTTAGA AAAGCAAATC 240
TAAAATTATC TGAAAAGGGA ATGAGAATAG TGAATGGACC AATAATAATG ACTAGAGAAG 300
AAAGAATGAA GATTGTTCAT GAAATTAAGG AACGAATATT GGATAAATAT GGGGATGATG 360
TTAAGGCTAT TGGTGTTTAT GGCTCTCTTG GTCGTCAGAC TGATGGGCCC TATTCGGATA 420
TTGAGATGAT GTGTGTCATG TCAACAGAGG AAGCAGAGTT CAGCCATGAA TGGACAACCG 480
GTGAGTGGAA GGTGGAAGTG AATTTTGATA GCGAAGAGAT TCTACTAGAT TATGCATCTC 540
AGGTGGAATC AGATTGGCCG CTTACACATG GTCAATTTTT CTCTATTTTG CCGATTTATG 600
ATTCAGGTGG ATACTTAGAG AAAGTGTATC AAACTGCTAA ATCGGTAGAA GCCCAAACGT 660
TCCACGATGC GATTTGTGCC CTTATCGTAG AAGAGCTGTT TGAATATGCA GGCAAATGGC 720
GTAATATTCG TGTGCAAGGA CCGACAACAT TTCTACCATC CTTGACTGTA CAGGTAGCAA 780
TGGCAGGTGC CATGTTGATT GGTCTGCATC ATCGCATCTG TTATACGACG AGCGCTTCGG 840
TCTTAACTGA AGCAGTTAAG CAATCAGATC TTCCTTCAGG TTATGACCAT CTGTGCCAGT 900
TCGTAATGTC TGGTCAACTT TCCGACTCTG AGAAACTTCT GGAATCGCTA GAGAATTTCT 960
GGAATGGGAT TCAGGAGTGG ACAGAACGAC ACGGATATAT AGTGGATGTG TCAAAACGCA 1020
TACCATTTTG AACGATGACC TCTAATAATT GTTAATCATG TTGGTTACGG GGATCCGTCG 1080
ACCTGCAGCC AAGCTTATCG ATTGCACTAT GCCTAAAACA ATTATACGAA AAGATCGCGA 1140
AACTTTCAAT ATACTCTGAT TATAATCCTC AAAACAGAAG GCCCGCACTC GCGGCAGGCC 1200
TTCATTTCCG CCGACCGATC ACCCCTTGGT TCCGCCGGCC GTCAACCCGG AAATCAGCCA 1260
TCGTTGGGCC GGAATTAGAT CTAGCATGCC TTTTAGTCCA GACCAAAATC CCTTAACGTG 1320
AGTTTTCGTT CCACTGAGCG TCAGACCCCT TAATAAGATG ATCTTCTTGA GATCGTTTTG 1380
GTCTGCGCGT AATCTCTTGC TCTGAAAACG AAAAAACCGC CTTGCAGGGC GGTTTTTCGA 1440
AGGTTCTCTG AGCTACCAAC TCTTTGAACC GAGGTAACTG GCTTGGAGGA GCGCAGTCAC 1500
CAAAACTTGT CCTTTCAGTT TAGCCTTAAC CGGCGCATGA CTTCAAGACT AACTCCTCTA 1560
AATCAATTAC CAGTGGCTGC TGCCAGTGGT GCTTTTGCAT GTCTTTCCGG GTTGGACTCA 1620
AGACGATAGT TACCGGATAA GGCGCAGCGG TCGGACTGAA CGGGGGGTTC GTGCATACAG 1680
TCCAGCTTGG AGCGAACTGC CTACCCGGAA CTGAGTGTCA GGCGTGGAAT GAGACAAACG 1740
CGGCCATAAC AGCGGAATGA CACCGGTAAA CCGAAAGGCA GGAACAGGAG AGCGCACGAG 1800
GGAGCCGCCA GGGGGAAACG CCTGGTATCT TTATAGTCCT GTCGGGTTTC GCCACCACTG 1860
ATTTGAGCGT CAGATTTCGT GATGCTTGTC AGGGGGGCGG AGCCTATGGA AAAACGGCTT 1920
TGCCGCGGCC CTCTCACTTC CCTGTTAAGT ATCTTCCTGG CATCTTCCAG GAAATCTCCG 1980
CCCCGTTCGT AAGCCATTTC CGCTCGCCGC AGTCGAACGA CCGAGCGTAG CGAGTCAGTG 2040
AGCGAGGAAG CGGAATATAT CCTGTATCAC ATATTCTGCT GACGCACCGG TGCAGCCTTT 2100
TTTCTCCTGC CACATGAAGC ACTTCACTGA CACCCTCATC AGTGCCAACA TAGTAAGCCA 2160
GTATACACTC CGCTGATTTC ACTTTTTGCA TTCTACGGAC TGCATAACTC ATATGTAAAT 2220
CGCTCCCTTT TAGGTGGCAC AAATGTGAGG CATTTTCGCT CTTTCCGGCG AGGCTAGTTA 2280
CCCTTAAGTT ATTGGTATGA CTGGTTTTAA GCGCAAAAAA AGTTGCTTTT TCGTACCTAT 2340
TAATCTATCG TTAGAAAACC GACTGTAAAA AGTACAGTCG GCATTATCTC ATATTATAAA 2400
 
AGCCAGTCAT TAGGCCTATC TGACAATTCC TGAATAGAGT TCATAAACAA TCCTGCATGA 2460
TAACCATCAC AAACAGAATG ATGTACCTGT AAAGATAGCG GTAAATATAT TGAATTACCT 2520
TTATTAATGA ATTTTCCTGC TGTAATAATG GGTAGAAGGT AATTACTATT ATTATTGATA 2580
TTTAAGTTAA ACCCAGTAAA TGAAGTCCAT GGAATAATAG AAAGAGAAAA AGCATTTTCA 2640
GGTATAGGTG TTTTGGGAAA CAATTTCCCC GAACCATTAT ATTTCTCTAC ATCAGAAAGG 2700
TATAAATCAT AAAACTCTTT GAAGTCATTC TTTACAGGAG TCCAAATACC AGAGAATGTT 2760
TTAGATACAC CATCAAAAAT TGTATAAAGT GGCTCTAACT TATCCCAATA ACCTAACTCT 2820
CCGTCGCTAT TGTAACCAGT TCTAAAAGCT GTATTTGAGT TTATCACCCT TGTCACTAAG 2880
AAAATAAATG CAGGGTAAAA TTTATATCCT TCTTGTTTTA TGTTTCGGTA TAAAACACTA 2940
ATATCAATTT CTGTGGTTAT ACTAAAAGTC GTTTGTTGGT TCAAATAATG ATTAAATATC 3000
TCTTTTCTCT TCCAATTGTC TAAATCAATT TTATTAAAGT TCATTTGATA TGCCTCCTAA 3060
ATTTTTATCT AAAGTGAATT TAGGAGGCTT ACTTGTCTGC TTTCTTCATT AGAATCAATC 3120
CTTTTTTAAA AGTCAATATT ACTGTAACAT AAATATATAT TTTAAAAATA TCCCACTTTA 3180
TCCAATATTC GTTCCTTAAT TTCATGAACA ATCTTCATTC TTTCTTCTCT AGTCATTATT 3240
ATTGGTCCCA GATCTGGTTG AACTACTCTT TAATAAAATA ATTTTTCCGT TCCCAATTCC 3300
ACATTGCAAT AATAGAAAAT CCATCTTCAT CGGCTTTTTC GTCATCATCT GTATGAATCA 3360
AATCGCCTTC TTCTGTGTCA TCAAGGTTTA ATTTTTTATG TATTTCTTTT AACAAACCAC 3420
CATAGGAGAT TAACCTTTTA CGGTGTAAAC CTTCCTCCAA ATCAGACAAA CGTTTCAAAT 3480
TCTTTTCTTC ATCATCGGTC ATAAAATCCG TATCCTTTAC AGGATATTTT GCAGTTTCGT 3540
CAATTGCCGA TTGTATATCC GATTTATATT TATTTTTCGG TCGAATCATT TGAACTTTTA 3600
CATTTGGATC ATAGTCTAAT TTCATTGCCT TTTTCCAAAA TTGAATCCAT TGTTTTTGAT 3660
TCACGTAGTT TTCTGTATTC TTAAAATAAG TTGGTTCCAC ACATAACAAT ACATGCATGT 3720
GCTGATTATA AGAATTATCT TTATTATTTA TTGTCACTTC CGTTGCACGC ATAAAACCAA 3780
CAAGATTTTT ATTAATTTTT TTATATTGCA TCATTCGGCG AAATCCTTGA GCCATATCTG 3840
ACAAACTCTT ATTTAATTCT TCGCCATCAT AAACATTTTT AACTGTTAAT GTGAGAAACA 3900
ACCAACGAAC TGTTGGCTTT TGTTTAATAA CTTCAGCAAC AACCTTTTGT GACTGAATGC 3960
CATGTTTCAT TGCTCTCCTC CAGTTGCACA TTGGACAAAG CCTGGATTTA CAAAACCACA 4020
CTCGATACAA CTTTCTTTCG CCTGTTTCAC GATTTTGTTT ATACTCTAAT ATTTCAGCAC 4080
AATCTTTTAC TCTTTCAGCC TTTTTAAATT CAAGAATATG CAGAAGTTCA AAGTAATCAA 4140
CATTAGCGAT TTTCTTTTCT CTCCATGGTC TCACTTTTCC ACTTTTTGTC TTGTCCACTA 4200
AAACCCTTGA TTTTTCATCT GAATAAATGC TACTATTAGG ACACATAATA TTAAAAGAAA 4260
CCCCCATCTA TTTAGTTATT TGTTTAGTCA CTTATAACTT TAACAGATGG GGTTTTTCTG 4320
TGCAACCAAT TTTAAGGGTT TTCAATACTT TAAAACACAT ACATAACAAC ACTTCAACGC 4380
ACCTTTCAGC AACTAAAATA AAAATGACGT TATTTCTATA TGTATCAAGA TAAGAAAGAA 4440
CAAGTTCAAA ACCATCAAAA AAAGACACCT TTTCAGGTGC TTTTTTTATT TTATAAACTC 4500
ATTGGGTGAT CTCGACTTCG TTCTTTTTTT ACCTCTCGGT TATGAGTTAG TTCAAATTCG 4560
TTCTTTTTAG GTTCTAAATC GTGTTTTTCT TGGAATTGTG CTGTTTTATC CTTTACCTTG 4620
TCTACAAACC CCTTAAAAAC GTTTTTAAAG GCTTTTAAGC CGTCTGTACG TTCCTTAAG 4679
(2) INFORMATION FOR SEQ ID NO 8
   (i) SEQUENCE CHARACTERISTICS
      (A) LENGTH 25 amino acids
      (B) TYPE amino acid
      (D) TOPOLOGY linear
   (ii) MOLECULE TYPE protein
   (xi) SEQUENCE DESCRIPTION SEQ ID NO 8 Met Asn Ile Lys Lys Leu Thr Pro Leu Leu Thr Leu Leu Leu Phe Phe 
1                    5                    10                    15

Ile Val Leu Ala Ser Pro Val Ser Ala
            20                    25
(2) INFORMATION FOR SEQ ID NO 9
   (i) SEQUENCE CHARACTERISTICS
      (A) LENGTH 435 amino acids
      (B) TYPE amino acid
      (D) TOPOLOGY linear
   (ii) MOLECULE TYPE protein
   (xi) SEQUENCE DESCRIPTION SEQ ID NO 9 
Ala Lys Tyr Leu Glu Leu Glu Glu Gly Gly Val Ile Met Gln Ala Phe
1               5                   10                  15

Tyr Trp Asp Val Pro Gly Gly Gly Ile Trp Trp Asp His Ile Arg Ser
            20                  25                  30

Lys Ile Pro Glu Trp Tyr Glu Ala Gly Ile Ser Ala Ile Trp Leu Pro
        35                  40                  45

Pro Pro Ser Lys Gly Met Ser Gly Gly Tyr Ser Met Gly Tyr Asp Pro
    50                  55                  60

Tyr Asp Tyr Phe Asp Leu Gly Glu Tyr Tyr Gln Lys Gly Thr Val Glu
65                  70                  75                  80

Thr Arg Phe Gly Ser Lys Glu Glu Leu Val Arg Leu Ile Gln Thr Ala
                85                  90                  95

His Ala Tyr Gly Ile Lys Val Ile Ala Asp Val Val Ile Asn His Arg
            100                 105                 110

Ala Gly Gly Asp Leu Glu Trp Asn Pro Phe Val Gly Asp Tyr Thr Trp
        115                 120                 125

Thr Asp Phe Ser Lys Val Ala Ser Gly Lys Tyr Thr Ala Asn Tyr Leu
    130                 135                 140

Asp Phe His Pro Asn Glu Leu His Cys Cys Asp Glu Gly Thr Phe Gly
145                 150                 155                 160

Gly Phe Pro Asp Ile Cys His His Lys Glu Trp Asp Gln Tyr Trp Leu
                165                 170                 175

Trp Lys Ser Asn Glu Ser Tyr Ala Ala Tyr Leu Arg Ser Ile Gly Phe
            180                 185                 190
 
Asp Gly Trp Arg Phe Asp Tyr Val Lys Gly Tyr Gly Ala Trp Val Val
        195                 200                 205

Arg Asp Trp Leu Asn Trp Trp Gly Gly Trp Ala Val Gly Glu Tyr Trp
    210                 215                 220

Asp Thr Asn Val Asp Ala Leu Leu Ser Trp Ala Tyr Glu Ser Gly Ala
225                 230                 235                 240

Lys Val Phe Asp Phe Pro Leu Tyr Tyr Lys Met Asp Glu Ala Phe Asp
                245                 250                 255

Asn Asn Asn Ile Pro Ala Leu Val Tyr Ala Leu Gln Asn Gly Gln Thr
            260                 265                 270

Val Val Ser Arg Asp Pro Phe Lys Ala Val Thr Phe Val Ala Asn His
        275                 280                 285

Asp Thr Asp Ile Ile Trp Asn Lys Tyr Pro Ala Tyr Ala Phe Ile Leu
    290                 295                 300

Thr Tyr Glu Gly Gln Pro Val Ile Phe Tyr Arg Asp Phe Glu Glu Trp
305                 310                 315                 320

Leu Asn Lys Asp Lys Leu Ile Asn Leu Ile Trp Ile His Asp His Leu
                325                 330                 335

Ala Gly Gly Ser Thr Thr Ile Val Tyr Tyr Asp Asn Asp Glu Leu Ile
            340                 345                 350

Phe Val Arg Asn Gly Asp Ser Arg Arg Pro Gly Leu Ile Thr Tyr Ile
        355                 360                 365

Asn Leu Ser Pro Asn Trp Val Gly Arg Trp Val Tyr Val Pro Lys Phe
    370                 375                 380

Ala Gly Ala Cys Ile His Glu Tyr Thr Gly Asn Leu Gly Gly Trp Val
385                 390                 395                 400

Asp Lys Arg Val Asp Ser Ser Gly Trp Val Tyr Leu Glu Ala Pro Pro
                405                 410                 415

His Asp Pro Ala Asn Gly Tyr Tyr Gly Tyr Ser Val Trp Ser Tyr Cys
            420                 425                 430

Gly Val Gly
        435
(2) INFORMATION FOR SEQ ID NO 10
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 10 
Ala Lys Tyr Leu Glu Leu Glu Glu Gly Gly
1               5                   10
(2) INFORMATION FOR SEQ ID NO 11
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 11 
Val Ile Met Gln Ala Phe Tyr Trp Asp Val
1               5                   10
(2) INFORMATION FOR SEQ ID NO 12
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 12 
Pro Gly Gly Gly Ile Trp Trp Asp His Ile
1               5                   10
(2) INFORMATION FOR SEQ ID NO 13
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 13 
Arg Ser Lys Ile Pro Glu Trp Tyr Glu Ala
1               5                   10
(2) INFORMATION FOR SEQ ID NO 14
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 14 
Gly Ile Ser Ala Ile Trp Leu Pro Pro Pro
1               5                   10
(2) INFORMATION FOR SEQ ID NO 15
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 15 
Ser Lys Gly Met Ser Gly Gly Tyr Ser Met
1               5                   10
(2) INFORMATION FOR SEQ ID NO 16
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 16 
Gly Tyr Asp Pro Tyr Asp Tyr Phe Asp Leu
1               5                   10
(2) INFORMATION FOR SEQ ID NO 17
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 17 
Gly Glu Tyr Tyr Gln Lys Gly Thr Val Glu
1               5                   10
(2) INFORMATION FOR SEQ ID NO 18
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 18 
Thr Arg Phe Gly Ser Lys Glu Glu Leu Val
1               5                   10
(2) INFORMATION FOR SEQ ID NO 19
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 19 
Arg Leu Ile Gln Thr Ala His Ala Tyr Gly
1               5                   10
(2) INFORMATION FOR SEQ ID NO 20
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 20 
Ile Lys Val Ile Ala Asp Val Val Ile Asn
1               5                   10
(2) INFORMATION FOR SEQ ID NO 21
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 21 
His Arg Ala Gly Gly Asp Leu Glu Trp Asn
1               5                   10
(2) INFORMATION FOR SEQ ID NO 22
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 22 
Pro Phe Val Gly Asp Tyr Thr Trp Thr Asp
1               5                   10
(2) INFORMATION FOR SEQ ID NO 23
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 23 
Phe Ser Lys Val Ala Ser Gly Lys Tyr Thr
1               5                   10
(2) INFORMATION FOR SEQ ID NO 24
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 24 
Ala Asn Tyr Leu Asp Phe His Pro Asn Glu
1               5                   10
(2) INFORMATION FOR SEQ ID NO 25
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 25 
Leu His Cys Cys Asp Glu Gly Thr Phe Gly
1               5                   10
(2) INFORMATION FOR SEQ ID NO 26
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 26 
Gly Phe Pro Asp Ile Cys His His Lys Glu
1               5                   10
(2) INFORMATION FOR SEQ ID NO 27
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 27 
Trp Asp Gln Tyr Trp Leu Trp Lys Ser Asn
1               5                   10
(2) INFORMATION FOR SEQ ID NO 28
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 28 
Glu Ser Tyr Ala Ala Tyr Leu Arg Ser Ile
1               5                   10
(2) INFORMATION FOR SEQ ID NO 29
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 29 
Gly Phe Asp Gly Trp Arg Phe Asp Tyr Val
1               5                   10
(2) INFORMATION FOR SEQ ID NO 30
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 30 
Lys Gly Tyr Gly Ala Trp Val Val Arg Asp
1               5                   10
(2) INFORMATION FOR SEQ ID NO 31
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 31 Trp Leu Asn Trp Trp Gly Gly Trp Ala.Val 1                    5                         10
(2) INFORMATION FOR SEQ ID NO 32
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 32 
Gly Glu Tyr Trp Asp Thr Asn Val Asp Ala
1               5                   10
(2) INFORMATION FOR SEQ ID NO 33
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 33 
Leu Leu Ser Trp Ala Tyr Glu Ser Gly Ala
1               5                   10
(2) INFORMATION FOR SEQ ID NO 34
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 34 
Lys Val Phe Asp Phe Pro Leu Tyr Tyr Lys
1               5                   10
(2) INFORMATION FOR SEQ ID NO 35
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 35 
Met Asp Glu Ala Phe Asp Asn Asn Asn Ile
1               5                   10
(2) INFORMATION FOR SEQ ID NO 36
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 36 
Pro Ala Leu Val Tyr Ala Leu Gln Asn Gly
1               5                   10
(2) INFORMATION FOR SEQ ID NO 37
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 37 
Gln Thr Val Val Ser Arg Asp Pro Phe Lys
1               5                   10
(2) INFORMATION FOR SEQ ID NO 38
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 38 
Ala Val Thr Phe Val Ala Asn His Asp Thr
1               5                   10
(2) INFORMATION FOR SEQ ID NO 39
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 39 
Asp Ile Ile Trp Asn Lys Tyr Pro Ala Tyr
1               5                   10
(2) INFORMATION FOR SEQ ID NO 40
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 40 
Ala Phe Ile Leu Thr Tyr Glu Gly Gln Pro
1               5                   10
(2) INFORMATION FOR SEQ ID NO 41
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 41 
Val Ile Phe Tyr Arg Asp Phe Glu Glu Trp
1               5                   10
(2) INFORMATION FOR SEQ ID NO 42
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 42 
Leu Asn Lys Asp Lys Leu Ile Asn Leu Ile
1               5                   10
(2) INFORMATION FOR SEQ ID NO 43
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 43 
Trp Ile His Asp His Leu Ala Gly Gly Ser
1               5                   10
(2) INFORMATION FOR SEQ ID NO 44
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 44 
Thr Thr Ile Val Tyr Tyr Asp Asn Asp Glu
1               5                   10
(2) INFORMATION FOR SEQ ID NO 45
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 45 
Leu Ile Phe Val Arg Asn Gly Asp Ser Arg
1               5                   10
(2) INFORMATION FOR SEQ ID NO 46
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 46 
Arg Pro Gly Leu Ile Thr Tyr Ile Asn Leu
1               5   .               10
(2) INFORMATION FOR SEQ ID NO 47
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 47 
Ser Pro Asn Trp Val Gly Arg Trp Val Tyr
1               5                   10
(2) INFORMATION FOR SEQ ID NO 48
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 48 
Val Pro Lys Phe Ala Gly Ala Cys Ile His
1               5                   10
(2) INFORMATION FOR SEQ ID NO 49
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 49 
Glu Tyr Thr Gly Asn Leu Gly Gly Trp Val
1               5                   10
(2) INFORMATION FOR SEQ ID NO 50
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 50 
Asp Lys Arg Val Asp Ser Ser Gly Trp Val
1               5                   10
(2) INFORMATION FOR SEQ ID NO 51
   (i) SEQUENCE CHARACTERISTICS
      (A) LENGTH 10 amino acids
      (B) TYPE amino acid
      (D) TOPOLOGY linear
   (ii) MOLECULE TYPE protein
   (xi) SEQUENCE DESCRIPTION SEQ ID NO 51 
Tyr Leu Glu Ala Pro Pro His Asp Pro Ala
1               5                   10
(2) INFORMATION FOR SEQ ID NO 52
   (i) SEQUENCE CHARACTERISTICS
      (A) LENGTH 15 amino acids
      (B) TYPE amino acid
      (D) TOPOLOGY linear
   (ii) MOLECULE TYPE protein
   (xi) SEQUENCE DESCRIPTION SEQ ID NO 52

Asn Gly Tyr Tyr Gly Tyr Ser Val Trp Ser Tyr Cys Gly Val Gly
1 . 5 10 15